# EUROPEAN PATENT SPECIFICATION

(11) **EP 1 117 375 B1**
(45) Date of publication and mention of the grant of the patent: **14.06.2006**
(21) Application number: 99938083.5
(22) Date of filing: 04.08.1999
(51) Int. Cl.: A61K 8/97, A61K 36/00, A61Q 7/00, C11D 3/382

(54) **TOPICAL LOTION CONTAINING OATSTRAW**
HAFESTROH ENTHALTENDE TOPISCHE LOTION
LOTION A ACTION LOCALE, A BASE DE PAILLE D'AVOINE

(30) Priority: 05.08.1998 US 95440 P
(43) Date of publication of application: 25.07.2001
(73) Proprietor: Mignault, Lorraine, Winnipeg, Manitoba R3P 2A4 (CA)
(72) Inventor: Mignault, Lorraine, Winnipeg, Manitoba R3P 2A4 (CA)
(74) Representative: Pratt, David Martin
(86) International application number: PCT/CA1999/000735
(87) International publication number: WO 2000/007556

(56) References cited:
- EP-A- 0 432 354
- EP-A- 0 661 047
- EP-A- 0 739 621
- FR-A- 2 762 515
- US-A- 5 620 695

## Description

### BACKGROUND OF THE INVENTION

At present, there is a trend in society away from chemical or synthetic pain relievers due to growing concerns about side-effects, long-term effects and dependency. However, individuals continue to experience the aches and pains associated with daily life, such as, scrapes, cuts, muscle pulls, spasms and sprains. Furthermore, there are also many individuals who suffer front pain, swelling and/or inflammation caused by conditions such as psoriasis, shingles, boils, cold sores, bums, sunburns, menstrual bloating, menstrual cramps, foot pain, acne, eczema, rosacea, dermatitis, insect bites, cancer treatments and arthritts, to name a few. Clearly, a lotion composed of natural ingredients that could be used to treat all of these ailments is needed.

### SUMMARY OF THE INVENTION

According to a first aspect of the invention, there is provided a topical lotion for relieving pain, swelling or inflammation comprising:
the active ingredient consisting of oatstraw extract; and
water,
characterized in that the oatstraw extract is prepared by steeping oatstraw in water.

The topical lotion may consist essentially of:
at least 50% oatstraw extract:
at least 25% glycerine; and
0.1-0.2% lavender oll,
the sum of these three components being 100%.

Alternatively, the topical lotion may consist essentially of:
0.1% lavender oil;
25% vegetable glycerine;
and q.s, to 100% filtered oatstraw extract in filtered and magnetized water.

According to a second aspect of the invention, there is provided a method of treating pain, swelling, itching or inflammation comprising:
providing a topical lotion the lotion consisting essentially of:
   at least 50% oatstraw extract;
   at least 25% glycerine; and
   0.1-0.2% lavender oil,
   the sum of these three components being 100%; and
   applying the lotion topologically to inflamed, painful or swollen areas.

The pain, swelling, itching or inflammation may be caused by a condition selected from one of the following: psoriasis: leprosy, skin poisoning from plants, shingles measles; chicken pox: boils: cold sores: coids and flu: sinus congestion; sun damage; bums; sunbums: menstrual bloating: menstrual cramps: foot pain: acne; eczama; rosacea: dermatitis; insect bites: parasitic infections; herniated discs; back and/or leg spasms; sore or damaged muscles, ligaments and tendons; bruising: varicose veins; fibromyalgia: multiple sclerosis; cancer treatments: internal organ injuries; brain and nerve surgery; and arthritis.

According to a third aspect of the invention, there is provided a method of preventing hair loss comprising:
providing a topical lotion the lotion consisting essentially of:
   at least 50% oatstraw extract
   at least 25% glycerine; and
   0.1-0.2% lavender oil.
   the sum of these three components being 100%; and
   applying the topical lotion to the scalp of an individual, thereby stopping hair loss.

According to a fourth aspect of the invention, there is provided a method of promoting hair growth comprising:
providing a topical lotion consisting essentially of:
   at least 50% oatstraw extract;
   at least 25% glycerine; and
   0.1-0.2% lavender oil,
   the sum of these three components being 100%; and
   applying the topical lotian to the scalp of an individual, thereby promoting hair growth.

According to a fifth aspect of the invention, there is provided a method of relieving headache pain comprising:
providing a topical lotion consisting essentially of:
   at least 50% oatstraw extract;
   at least 25% glycerine; and
   0.1-0.2% lavender oil,
   the sum of these three components being 100%; and
   applying the lotion topologically to the forehead and neck of the individual, thereby relieving headache pain.

According to a sixth aspect of the invention, there is provided a facial cleanser comprising:
a lotion consisting essentially of:
   at least 50% oatstraw extract;
   at least 25% glycerine; and
   0.1-0.2% lavender oil,
   the sum of these three components being 100%; and
   a suitable carrier.

According to a seventh aspect of the Invention, there is provided a cosmetic composition comprising:
a lotion consisting essentially of:
   at least 50% oatstraw extract;
   at least 25% glycerine, and
   0.1-0.2% lavender oil,
   the sum of these three components being 100%; and
   a suitable carrier.

According to a eighth aspect of the invention, there is provided a method of inducing a deeper sleep in an individual comprising:
providing a topical lotion consisting essentially of:
   at least 50% oatstraw extract;
   at least 25% glycerine; and
   0.1-0.2% lavender oil,
   the sum of these three components being 100%; and
   applying the lotion topologically to the soles of the feet of the individual.

According to an ninth aspect of the invention, there is provided a laundry additive comprising:
a mixture consisting essentially of:
   at least 50% oatstraw extract;
   at least 25% glycerine; and
   0.1-0.2% lavender oil,
   the sum of these three components being 100%; and
   a suitable carrier.

According to a tenth aspect of the invention, there is provided a hair or body product comprising:
a mixture consisting essentially of:
   at least 50% oatstraw extract;
   at feast 25% glycerine; and
   0.1-0.2% lavender oil,
   the sum of these three components being 100%; and
   a suitable carrier.

According to a eleventh aspect of the invention, there is provided a process for preparing a topical lotion comprising:
providing a quantity of oatstraw;
providing heated water,
placing the oatstraw in the heated water,
steeping the oatstraw in the heated water, thereby producing an oststraw mixture;
flitering the oatstraw mixture, thereby produang an oatstraw extract; and
mixing the oatstraw extract with glycerine and lavender oil, thereby producing a topical lotion.

The topical lotion may consist essentially of:
at least 50% oatstraw extract,
at least 25% glycerine; and
0.1-0.2% lavender oil,
the sum of these three components being 100%.

### DETAILED DESCRIPTION

The present invention is described In detail based on examples; however, the invention is not limited to the examples.

### FORMULA EXAMPLE 1

50-75% catstraw concentrate;
25-50% glycerine; and
0.1-0.2% lavender oil.

### FORMULA EXAMPLE II

75% filtered oatstraw concentrate;
25% vegetable glycerine; and
0.1 % lavender oil.

The oatstraw concentrate is prepared by steeping 5 grams of oatstraw in 4 cups of water (940 ml). In this embodiment, the oatstraw is consumer ready oatstraw and the water is filtered/magnetized water. It is of note that the oatstraw may be blended and concentrated first to facilitate steeping.

Specifically, the water is heated in a stainless steel container until hot and tiny bubbles have formed on the bottom of the container. The dry oatstraw is placed in a heat resistant glass container and the heated water is added to the oatstraw. The oatstraw is mixed into the water and the heat resistant glass container is covered. The mixture is then steeped for approximately 2 hours. During this time, the oatstraw mixture becomes golden yellow in colour. The oatstraw mixture is then filtered and the filtered solution is the oatstraw concentrate.

The oatstraw concentrate is then mixed with the glycerine and the lavender oil as described above. In this embodiment, the glycerine is vegetable glycerine, although other sources may also be used. The resulting mixture is then aliquoted into coloured bottles and stored until needed.

For use, a dropper may be used to remove a quantity of the mixture from a given bottle and used to dispense drops of the mixture onto the afflicted area. The mixture is then rubbed topologically into the afflicted area, as described below.

In use, topological application of the lotion promotes healing and well-being of an injured area. As a result, the lotion has a variety of uses and applications.

For example, the lotion promotes muscle relaxation and healing. Specifically, topological application of the lotion to an injured area facilitates chiropractic adjustments, physiotherapy and massage therapy.

Furthermore, application of the lotion stops inflammation, swelling, pain and/or itching caused by insect bites as well as skin conditions such as, for example, eczema, dermatitis, psoriasis, skin poisoning from plants, shingles, chicken pox; measles; boils, cold sores, acne, rosacea and the like. In addition, the lotion may be used to treat razor burn or nicks or cuts that occur during shaving or sun damage. Furthermore, the above-described lotion also acts as a pre-bite treatment, as topical application of the lotion reduces severity of subsequent insect bites.

The lotion also provides relief from pain and inflammation resulting from burns, such as, for example, sunburns, scalds, radiation bums and mechanical burns as well as pain and inflammation caused by some cancer treatments.

Application of the lotion has also been shown to provide relief from pain and inflammation caused by other ailments, such as, for example, arthritis, herniated discs, back and/or leg spasms, foot pain, burning feet (caused by kidney disease); internal organ injuries; varicose veins; fibromyaglia; multiple sclerosis; sinus congestion; colds and flu; parasitic infections; menstrual bloating and/or cramping as well as sore or damaged muscles, ligaments and tendons.

Application of the lotion promotes capillary repair, for example, fine blood vessels, which in turn means that the lotion can be used to dissipate discoloration caused by bruising and promote healing of bruises.

Furthermore, application of the lotion to the frontal area of the skull, the base of the skull or the neck area provides relief from headaches and tension.

As noted above, the lotion has been shown to be effective in the treatment of psoriasis. Specifically, the lotion has been shown to prevent hair loss and actually promotes hair re-growth of individuals having psoriasis of the scalp or hair loss caused by diseases such as for example German measles. Thus, in another embodiment of the invention, the lotion is applied topologically to the scalp of an individual to prevent hair loss and promote hair re-growth.

In another embodiment of the invention, the lotion is used to promote deeper, more restful sleep by applying the lotion directly to the soles of the feet of an individual, thereby effecting the receptors located on the soles of the feet and allowing the user to experience a deeper, more restful sleep. Furthermore, application of the lotion in this manner has also been shown to enhance sleep during pregnancy while also controlling nausea.

In yet another embodiment of the invention, the lotion may be combined with suitable carriers and other elements known in the art of cosmetics to produce creams and lotions suitable for use for general skin care. The lotion can be used for cleansing and toning skin, reducing cellulite, removing excess oil and/or sebum, balancing oily and dry skin patches, improving skin texture and smoothness, removing small facial skin bumps and skin tags, refining pore size, facilitating eye and/or lip make-up application and wearability, enhancing skin colour, evening out skin discolorations as well as using the combination as an anti-wrinkling agent.

In another embodiment of the invention, the above-described lotion is combined with carriers known in the art to produce hair and body cleaning products and the like, for example, body washes; shaving creams; shaving gels; shaving lotions; shampoos; conditioners; body lotions; moisturizing lotions; facial and wrinkle lotions; hand lotions; body creams; hand creams; facial creams; after-shave lotions; skin cleansing preparations; make-up removers; personal deodorants; suntan oil preparations; sunscreen preparations; sun block preparations; lip balms; aromatherapy products; massage gels; foot lotions; facial masques; pimple/acne preparations; facial and body firmers; pore size reducing preparations; styling lotions; and styling sprays.

In another embodiment of the invention, the lotion is combined with a suitable carrier and used to remove stains in clothing without damaging the fibres by applying the lotion directly to the stained area of the clothing. In addition, the lotion acts as a bleach by brightening white and coloured clothing. The lotion also acts as a fabric softener. Furthermore, the above-described combination rinses easily and produces a relatively low level of suds, meaning that it is very enviro-friendly.

In yet another embodiment of the invention, the above-described lotion is used as a carrier for pharmaceutical compositions, thereby taking advantage of the fact that the lotion is readily absorbed by the skin.

It is of note that the above-described lotion may also be used in applications for animal or veterinary products similar to those described above.

Thus, as described above, the above-described lotion has many applications and uses.

## Claims

1. A topical lotion for relieving pain, swelling or inflammation, the lotion comprising:
an active ingredient consisting of oatstraw extract; and
heated water,
**characterised in that** the oatstraw extract is prepared by steeping oatstraw in the heated water and filtering the oatstraw.

2. A topical lotion according to claim 1, wherein the water is magnetised water.

3. A topical lotion according to claim 2, further comprising glycerine.

4. A topical lotion according to claim 3, further comprising lavender oil.

5. A topical lotion according to claim 4, wherein the glycerine is vegetable glycerine.

6. A topical lotion according to claim 4, wherein the topical lotion consists essentially of:
at least 50% oatstraw extract;
at least 25% glycerine; and
0.1-0.2% lavender oil,
the sum of these three components being 100%.

7. A topical lotion according to claim 5, wherein the topical lotion consists essentially of:
0.1% lavender oil;
25% vegetable glycerine; and
q.s. to 100% filtered oatstraw extract in filtered and magnetised water.

8. A topical lotion according to any one of claims 1 to 7, for use in treating pain, swelling, itching or inflammation.

9. A topical lotion according to claim 8, wherein the pain, swelling, itching or inflammation is caused by a condition selected from one of the following: psoriasis; leprosy; skin poisoning from plants, shingles; measles; chicken pox; boils; cold sores; colds and flu; sinus congestion; sun damage; burns; sunburns; menstrual bloating; menstrual cramps; foot pain; acne; eczema; rosacea; dermatitis; insect bites; parasitic infections; herniated discs; back and/or leg spasms; sore or damaged muscles, ligaments and tendons; bruising; varicose veins; fibromyalgia; multiple sclerosis; cancer treatments; internal organ injuries; brain and nerve surgery; and arthritis.

10. A non-therapeutic method of preventing hair loss comprising:
applying a topical lotion according to claim 6 to the scalp of an individual.

11. A non-therapeutic method of promoting hair growth comprising applying a topical lotion according to claim 6 to the scalp of the individual.

12. Use of a composition according to claim 6 for the preparation of a medicament for the prevention of hair loss caused by psoriasis.

13. Use of a composition according to claim 6 for the preparation of a medicament for promoting hair growth.

14. A topical lotion according to claim 6 for use in relieving headache pain.

15. A facial cleanser comprising:
a topical lotion according to claim 6; and
a suitable carrier.

16. A cosmetic composition comprising:
a topical lotion according to claim 6; and
a suitable carrier.

17. A topical lotion according to claim 6, for use in inducing a deeper sleep in an individual.

18. A hair or body product comprising:
a topical lotion according to claim 6; and
a suitable carrier.

19. A product according to claim 18, selected from the group consisting of: body washes; shaving creams; shaving gels; shaving lotions; shampoos; conditioners; body lotions; moisturising lotions; facial and wrinkle lotions; hand lotions; body creams; hand creams; facial creams; after-shave lotions; skin cleansing preparations; make-up removers; personal deodorants; suntan oil preparations; sunscreen preparations; sun block preparations; lip balms; aromatherapy products; massage gets; foot lotions; facial masques; pimple/acne preparations; facial and body firmers; pore size reducing preparations; styling lotions; and styling sprays.

20. A process for preparing a topical lotion, the process comprising:
providing a quantity of oatstraw;
providing heated water;
placing the oatstraw in the heated water;
steeping the oatstraw in the heated water, thereby producing an oatstraw mixture;
filtering the oatstraw mixture, thereby producing an oatstraw extract; and
mixing the oatstraw extract with glycerine and lavender oil, thereby producing a topical lotion.

21. A process according to claim 20, wherein the heated water is magnetised water.

22. A process according to claim 21, wherein the topical lotion consists essentially of:
at least 50% oatstraw extract;
at least 25% glycerine; and
0.1-0.2% lavender oil,
the sum of these three components being 100%.

## Patentansprüche

1. Topische Lotion zum Lindern von Schmerz, Schwellung oder Entzündung, wobei die Lotion umfasst:
einen aktiven Bestandteil bestehend aus Haferstrohextrakt; und
heißes Wasser,
**dadurch gekennzeichnet, dass** der Haferstrohextrakt hergestellt wird durch Einweichen von Haferstroh in dem heißen Wasser und Abfiltrieren des Haferstrohs.

2. Topische Lotion gemäß Anspruch 1, wobei das Wasser magnetisiertes Wasser ist.

3. Topische Lotion gemäß Anspruch 2, die zusätzlich Glycerin umfasst.

4. Topische Lotion gemäß Anspruch 3, die zusätzlich Lavendelöl umfasst.

5. Topische Lotion gemäß Anspruch 4, wobei das Glycerin pflanzliches Glycerin ist.

6. Topische Lotion gemäß Anspruch 4, wobei die topische Lotion im Wesentlichen besteht aus:
mindestens 50% Haferstrohextrakt;
mindestens 25% Glycerin; und
0,1% bis 0,2% Lavendelöl,
wobei die Summe dieser drei Bestandteile 100% ergibt.

7. Topische Lotion gemäß Anspruch 5, wobei die topische Lotion im Wesentlichen besteht aus:
0, 1 % Lavendelöl;
25% pflanzlichem Glycerin; und
Rest bis 100% filtrierter Haferstrohextrakt in filtriertem und magnetisiertem Wasser.

8. Topische Lotion gemäß einem der Ansprüche 1 bis 7 zur Verwendung in der Behandlung von Schmerzen, Schwellung, Juckreiz oder Entzündung.

9. Topische Lotion gemäß Anspruch 8, wobei der Schmerz, die Schwellung, der Juckreiz oder die Entzündung hervorgerufen werden durch ein Leiden, ausgewählt aus den folgenden: Psoriasis; Lepra; Hautvergiftung durch Pflanzen; Gürtelrose; Masern, Windpocken; Geschwüre; Gesichtsherpes; Erkältungen und Grippe; Sinuskongestion; Sonnenschädigungen; Verbrennungen; Sonnenbrand; Menstruationsschwellung; Menstruationskrämpfe; Fußschmerz; Akne; Ekzem; Rosacea; Dermatitis; Insektenstiche; Parasiteninfektionen; Bandscheibenvorfall; Rücken- und/oder Beinspasmen; entzündete oder beschädigte Muskeln, Bänder und Sehnen; Prellung; Krampfadern; Fibromyalgie; Multiple Sklerose; Krebsbehandlungen; innere Organverletzungen; chirurgischer Eingriff an Gehirn und Nerven; und Arthritis.

10. Nicht-therapeutisches Verfahren zum Verhindern von Haarausfall, umfassend:
Auftragen einer topischen Lotion gemäß Anspruch 6 auf die Kopfhaut eines Individuums.

11. Nicht-therapeutisches Verfahren zum Fördern des Haarwachstums, umfassend:
Auftragen einer topischen Lotion gemäß Anspruch 6 auf die Kopfhaut eines Individuums.

12. Verwendung einer Zusammensetzung gemäß Anspruch 6 zur Herstellung eines Medikaments zum Verhindern von Haarausfall, der durch Psoriasis hervorgerufen wird.

13. Verwendung einer Zusammensetzung gemäß Anspruch 6 zur Herstellung eines Medikaments zum Fördern von Haarwachstum.

14. Topische Lotion gemäß Anspruch 6 zur Verwendung bei der Linderung von Kopfschmerz.

15. Gesichtsreinigungscreme, umfassend:
eine topische Lotion gemäß Anspruch 6; und
einen geeigneten Träger.

16. Kosmetische Zusammensetzung, umfassend:
eine topische Lotion gemäß Anspruch 6; und
einen geeigneten Träger.

17. Eine topische Lotion gemäß Anspruch 6 zur Verwendung beim Einleiten eines tieferen Schlafs bei einem Individuum.

18. Haar- oder Körperprodukt, umfassend:
eine topische Lotion gemäß Anspruch 6;
einen geeigneten Träger.

19. Ergebnis gemäß Anspruch 18, ausgewählt aus der Gruppe, bestehend aus: Körperreinigungen; Rasiercremes, Rasiergels; Rasierlotionen; Shampoos; Conditionem; Körperlotionen; Feuchtigkeitslotionen; Gesichts- und Faltenlotionen; Handlotionen; Körpercremes; Handcremes; Gesichtscremes; Aftershavelotionen; Hautreinigungszubereitungen; Make-up-Entfemer; persönlichen Deodorants; Sonneölzubereitungen; Sonnenschutzzubereitungen; Sonnenblockerzubereitungen; Lippenbalsams; Aromatherapieprodukten; Massagegels; Fußlotionen; Gesichtsmasken; Pickel/Aknezubereitungen; Gesichts- und Körperstraffer; Zubereitung zur Verminderung der Porengröße; Formgebungslotionen; und Formgebungssprays.

20. Verfahren zur Herstellung einer topischen Lotion, wobei das Verfahren umfasst:
Bereitstellen einer Menge an Haferstroh;
Bereistellen von heißem Wasser;
Einbringen des Haferstrohs in das heiße Wasser;
Einweichen des Haferstrohs in dem heißen Wasser, wodurch eine Haferstrohmischung hergestellt wird;
Abfiltrieren der Haferstrohmischung, wodurch ein Haferstrohextrakt hergestellt wird; und
Vermischen des Haferstrohextraktes mit Glycerin und Lavendelöl, wodurch eine topische Lotion hergestellt wird.

21. Verfahren gemäß Anspruch 20, wobei das heiße Wasser magnetisiertes Wasser ist.

22. Verfahren gemäß Anspruch 21, wobei die topische Lotion im Wesentlichen besteht aus:
mindestens 50% Haferstrohextrakt;
mindestens 25% Glycerin; und
0,1% bis 0,2% Lavendelöl, wobei die Summe dieser drei Bestandteile 100% ergibt.

## Revendications

1. Lotion à usage local pour soulager la douleur, le gonflement ou l'inflammation, la lotion comprenant :
. une substance active consistant en un extrait de paille d'avoine ; et
. de l'eau chaude,
**caractérisée en ce que** l'extrait de paille d'avoine est préparé en faisant tremper la paille d'avoine dans l'eau chaude et en filtrant la paille d'avoine.

2. Lotion à usage local selon la revendication 1, dans laquelle l'eau est de l'eau magnétisée.

3. Lotion à usage local selon la revendication 2, comportant en outre de la glycérine.

4. Lotion à usage local selon la revendication 3, comportant en outre de l'essence de lavande.

5. Lotion à usage local selon la revendication 4, dans laquelle la glycérine est de la glycérine végétale.

6. Lotion à usage local selon la revendication 4, dans laquelle la lotion à usage local consiste essentiellement en :
. au moins 50% d'extrait de paille d'avoine ;
. au moins 25% de glycérine ; et
. de 0,1 à 0,2% d'essence de lavande,
la somme de ces trois composants étant de 100%.

7. Lotion à usage local selon la revendication 5, dans laquelle la lotion à usage local consiste essentiellement en :
. 0,1% d'essence de lavande ;
. 25% de glycérine végétale ; et
. quantité suffisante pour 100% d'extrait de paille d'avoine filtré dans de l'eau filtrée et magnétisée.

8. Lotion à usage local selon l'une quelconque des revendications 1 à 7, utilisée pour le traitement de la douleur, de la tuméfaction, de la démangeaison ou de l'inflammation.

9. Lotion à usage local selon la revendication 8, dans laquelle la douleur, la tuméfaction, la démangeaison ou l'inflammation est provoquée par un état choisi parmi un des suivantes : psoriasis ; lèpre ; empoisonnement de la peau par des plantes ; zona ; rougeole ; varicelle ; furoncles ; herpès ; rhumes et grippe ; congestion des sinus ; dommages dus au soleil ; brûlures ; coups de soleil ; gonflement menstruel ; crampes menstruelles ; douleur des pieds ; acné ; eczéma ; acné rosacée ; dermatite ; morsures d'insecte ; infections parasites ; disques herniés ; crampes de dos et/ou de jambe ; muscles, ligaments et tendons endoloris ou endommagés ; ecchymose ; varices ; fibromyalgie ; sclérose en plaques ; traitements du cancer ; dommages internes d'organe ; chirurgie du cerveau et des nerfs ; et arthrite.

10. Procédé non-thérapeutique pour empêcher la perte de cheveux, comprenant l'application d'une lotion à usage local selon la revendication 6 sur le cuir chevelu d'une personne.

11. Procédé non-thérapeutique pour favoriser la pousse des cheveux, comprenant l'application d'une lotion à usage local selon la revendication 6 sur le cuir chevelu d'une personne.

12. Utilisation d'une composition selon la revendication 6 pour la préparation d'un médicament pour la prévention de la perte des cheveux provoquée par le psoriasis.

13. Utilisation d'une composition selon la revendication 6 pour la préparation d'un médicament favorisant la pousse des cheveux.

14. Lotion à usage local selon la revendication 6, utilisée pour soulager le mal de tête.

15. Nettoyant du visage comprenant :
. une lotion à usage local selon la revendication 6 ; et
. un support approprié.

16. Composition cosmétique comprenant :
. une lotion à usage local selon la revendication 6 ; et
. un support approprié.

17. Lotion à usage local selon la revendication 6, pour induire un sommeil plus profond chez une personne.

18. Produit pour les cheveux ou pour le corps comprenant :
. une lotion à usage local selon la revendication 6 ; et
. un support approprié.

19. Produit selon la revendication 18, choisi dans le groupement constitué par : les produits lavants pour le corps ; les crèmes à raser ; les gels de rasage ; les lotions de rasage ; les shampooings ; les revitalisants ; les lotions pour le corps ; les lotions hydratantes ; les lotions pour le visage et les rides ; les lotions pour les mains ; les crèmes pour le corps ; les crèmes pour les mains ; les crèmes pour le visage ; les lotions après-rasage ; les préparations pour le nettoyage de la peau ; les démaquillants ; les déodorants ; les préparations d'huile de bronzage ; les préparations de protection solaire ; les préparations d'écran total ; les baumes à lèvre ; les produits d'aromathérapie ; les gels de massage ; les lotions pour les pieds ; les masques pour le visage ; les préparations pour les boutons/l'acnée ; les raffermissants pour le visage et pour le corps ; les préparations pour la réduction de la taille des pores ; les lotions à coiffer ; et les pulvérisations de coiffure.

20. Procédé de préparation d'une lotion à usage local, la procédé comprenant :
. une certaine quantité de paille d'avoine
. de l'eau chaude ;
. mise de la paille d'avoine dans l'eau chaude ;
. trempage de la paille d'avoine dans l'eau chaude, produisant ainsi un mélange de paille d'avoine ;
. filtration du mélange de paille d'avoine, produisant ainsi un extrait de paille d'avoine et
. mélange de l'extrait de paille d'avoine avec de la glycérine et de l'essence de lavande, pour produire une lotion à usage local.

21. Procédé selon la revendication 20, dans lequel l'eau chaude est de l'eau magnétisée.

22. Procédé selon la revendication 21, dans lequel la lotion à usage local consiste essentiellement en :
. au moins 50% d'extrait de paille d'avoine ;
. au moins 25% de glycérine ; et
. de 0,1 à 0,2% d'essence de lavande,
la somme de ces trois composants étant de 100%.
